# EUROPEAN PATENT APPLICATION

(11) **EP 4 852 005 A1**
(43) Date of publication of application: **09.12.2026**
(21) Application number: 25925332.6
(22) Date of filing: 30.12.2025
(51) Int. Cl.: G16C 60/00

(54) **OPTIMAL COMPONENT DESIGN METHOD FOR TITANIUM ALLOY, AND METHOD FOR PREPARING TITANIUM ALLOY**

(30) Priority: 14.02.2025 CN 202510163707
(71) Applicant: Chengdu Advanced Metal Materials Industry Technology Research Institute Co., Ltd., Chengdu, Sichuan 610300 (CN)
(72) Inventor: LI, Kun, Chengdu, Sichuan 610300 (CN); ZHU, Yanlin, Chengdu, Sichuan 610300 (CN); NING, Zhen, Chengdu, Sichuan 610300 (CN); WU, Die, .Chengdu, Sichuan 610300 (CN)
(74) Representative: V.O.
(86) International application number: PCT/CN2025/147363
(87) International publication number: WO 2026/170876

(57) **Abstract**

The present invention relates to the technical field of materials. Provided are an optimal component design method for a titanium alloy, and a method for preparing a titanium alloy. The optimal component design method for a titanium alloy comprises: step a, acquiring component-related data and property-related data of a titanium alloy, and on the basis of the associated component-related data and property-related data, constructing a data set; step b: by means of the data set and a random forest algorithm, training a relationship model indicating a correspondence between components and properties of the titanium alloy, so as to obtain a trained relationship model; step c, by means of the trained relationship model, determining a property value corresponding to each component in a preset component population of the titanium alloy; and step d, setting a multi-property objective of the titanium alloy, determining a priority of each component on the basis of the multi-property objective and the property value corresponding to each component, performing a genetic operation on the component with the highest priority so as to generate a next-generation component, updating the component population, and returning to step c until a first condition is met, so as to obtain an optimal component combination that meets the multi-property objective. The solution of the present invention avoids a complicated test process, and reduces the complexity of determining a titanium-alloy component design.

## Description

This application claims the priority of Chinese Patent Application No.202510163707.2, filed with the China National Intellectual Property Administration on February 14, 2025, and titled with "OPTIMAL COMPONENT DESIGN METHOD FOR TITANIUM ALLOY, AND METHOD FOR PREPARING TITANIUM ALLOY", the disclosure of which is hereby incorporated by reference in its entirety.

### FIELD

The present disclosure relates to the field of material technology, in particular to a method for optimizing design of titanium alloy composition and a method for manufacturing a titanium alloy.

### BACKGROUND

Conventional methods of designing titanium alloy compositions rely on trial and error to determine the corresponding composition combinations of titanium alloys that meet different performance requirements. This approach requires a large number of experiments and comprehensive analysis of the experimental results, barely resulting in a roughly optimal combination that meets the corresponding performance requirements. In addition to the low accuracy of the resulting optimal combination, the entire process is time-consuming, costly and inefficient, making it difficult to meet the demands of modern industry for titanium alloys.

An existing published patent application with application number 202411199233.9 discloses a method of designing the composition of titanium alloys with high comprehensive performance. It comprises the construction of an initial dataset, and performing data screening through Pearson correlation screening, recursive elimination and feature weighting sorting to determine the combination of key material descriptors affecting properties of titanium alloy; then constructing data containing alloy composition, key material descriptors and alloy properties based on the above screening results, constructing a prediction model, establishing an XGBoost initial model of key features using a training set, and performing dual optimization on hyperparameters in the XGBoost initial model using Grid Search and K-Fold Cross Validation algorithms to obtain an improved XGBoost model; utilizing a test set to evaluate prediction accuracy of the improved XGBoost model and perform optimization design to obtain the optimal XGBoost prediction model; and further performing collaborative optimization on a plurality of alloy performances using the multi-objective optimization strategy based on a non-dominated sorting algorithm to obtain the titanium alloy composition with the optimal comprehensive performance, followed by the experimental verification and iterative optimization. As can be seen from the above, although this method avoids steps of conducting a large number of experiments and comprehensively analyzing experimental results, the entire process involves multiple data preparation and extensive data calculation processes, thus increasing the complexity of designing titanium alloy composition from a calculation perspective.

In summary, there is an urgent need for a method for designing titanium alloy composition that avoids steps of conducting a large number of experiments and comprehensively analyzing experimental results, as well as eliminating the need for multiple data preparation and data calculation processes.

### SUMMARY

Although the related art avoids steps of conducting a large number of experiments and comprehensively analyzing experimental results, the entire process involves multiple data preparation and extensive data calculation processes, thus increasing the complexity of designing titanium alloy composition from a calculation perspective. For these issues, the present disclosure provides a method for optimizing design of titanium alloy composition and a method for manufacturing a titanium alloy.

According to a first aspect of the present disclosure, provided is a method for optimizing design of titanium alloy composition, comprising
step a: acquiring composition-related data and performance-related data of a titanium alloy, constructing a dataset based on the composition-related data and the performance-related data associated therewith,
step b: training a relationship model for a correspondence between composition and performance of the titanium alloy using the dataset and a random forest algorithm until a relevant parameter of the relationship model is determined to obtain a trained relationship model,
step c: determining each performance value corresponding to each composition in a preset composition population of the titanium alloy by the trained relationship model,
step d: setting a multi-performance objective for the titanium alloy, determining a priority of each composition based on the multi-performance objective and each performance value corresponding to each composition, performing a genetic operation on a composition with a highest priority to generate a next-generation composition and updating the composition population and returning to step c until a first condition is reached to obtain an optimal composition combination meeting the multi-performance objective.

In some embodiments, the multi-performance objective for the titanium alloy includes a tensile strength of the titanium alloy being greater than or equal to 900 MPa and an elongation of the titanium alloy being greater than or equal to 20%.

In some embodiments, step d comprises:
comparing a contribution of each performance value corresponding to each composition to the multi-performance objective by a non-dominated sorting algorithm, calculating a crowding degree of each composition, and determining a priority of each composition based on a principle that under the same domination condition, a lower crowding degree corresponds to a higher priority.

In some embodiments, step d further comprises:
performing a crossover operation on a composition with a highest priority according to a formula of ${x}_{new}^{\left(k\right)} = \frac{1 + β}{2} {x}_{p} + \frac{1 - β}{2} {x}_{q}$,
wherein xₚ and x_{q} are two parent compositions, β is a crossover factor generated based on a random number, and X_{new}^{(k)} is a generated pre-mutation next-generation composition; and
performing a mutation operation on the pre-mutation next-generation composition according to a formula of ${x}_{new}^{\left(k\right)} = {x}_{new}^{\left(k\right)} + δ$,
wherein δ is a small perturbation for performing polynomial mutation, and ${x}_{new}^{\left(k\right)}$ is a generated next-generation composition.

In some embodiments, step d further comprises:
until a maximum number of iterations is reached, updating the composition population with a next-generation composition obtained in a final iteration and determining an optimal composition combination meeting the multi-performance objective based on the updated composition population.

In some embodiments, the preset composition population is generated by a random algorithm, and the composition population comprises a plurality of composition vectors Xᵢ represented by a formula of x*ᵢ* = [*x*ᵢ₁, *x*ᵢ₂, ... , *xᵢₙ*],
wherein a composition vector xᵢₙ represents an n-th element in the composition vector Xᵢ, and a sum of corresponding contents from xᵢ₁ to xᵢₙ is 1.

In some embodiments, step b comprises:
dividing the dataset into a training set and a testing set at a ratio of 2:1, training a relationship model for a correspondence between the composition and the performance of the titanium alloy using the training set and a random forest algorithm, optimizing the relationship model by a cross-validation and a grid search, determining a relevant parameter of the relationship model, verifying the accuracy of the relationship model using the testing set, to obtain the trained relationship model if the verification is passed.

In some embodiments, step a comprises: acquiring the composition-related data and the performance-related data of the titanium alloy, performing missing value processing, data format unification, and vectorization processing on the composition-related data and the performance-related data that are associated with each other, and constructing a dataset based on the processed data.

In some embodiments, the composition-related data of the titanium alloy includes the types and contents of alloying elements and the weights and contents of impurity elements. The alloying elements include aluminum, vanadium, molybdenum and tin, and the impurity elements include iron, oxygen, nitrogen and carbon.

In some embodiments, the relevant parameter of the relationship model includes the number of decision trees, a maximum depth, a minimum number of samples for split, a minimum number of samples required to be at a leaf node and a maximum number of features.

According to a second aspect of the present disclosure, provided is a method for manufacturing a titanium alloy, comprising preparing a plurality of raw materials according to the method for optimizing design of titanium alloy composition according to any one of items described above, pre-processing the plurality of raw materials, placing them into a smelting furnace, controlling a relevant parameter of a smelting process and then casting and shaping to obtain a titanium alloy.

The method for optimizing design of titanium alloy composition and the method for manufacturing a titanium alloy described above comprises acquiring composition-related data and performance-related data of a titanium alloy, constructing a dataset based on the composition-related data and the performance-related data associated therewith, training a relationship model representing a correspondence between composition and performance of the titanium alloy using the dataset and a random forest algorithm until a relevant parameter of the relationship model is determined to obtain a trained relationship model, then determining each performance value corresponding to each composition in a preset composition population of the titanium alloy by the trained relationship model, setting a multi-performance objective for the titanium alloy, determining a priority of each composition based on the multi-performance objective, performing a genetic operation on a composition with a highest priority to generate a next-generation composition and updating the composition population and then determining each performance value corresponding to each composition in a preset composition population of the titanium alloy by the trained relationship model until a first condition is reached to obtain an optimal composition combination meeting the multi-performance objective. The method of the present disclosure eliminates the need for a large number of experiments and comprehensive analysis of experimental data, thereby avoiding cumbersome experimental procedures. Meanwhile, it obviates the need for multiple data preparation and extensive data calculation processes, reducing the complexity of designing and determining titanium alloy composition via algorithms and improving both the efficiency and accuracy of designing titanium alloy composition.

In addition, the method for manufacturing a titanium alloy of the present disclosure can also achieve the technical effects described above, which will not be detailed herein.

### BRIEF DESCRIPTION OF DRAWINGS

To more clearly illustrate technical solutions in the embodiments of the present disclosure or in the conventional technology, drawings required for describing the embodiments or the conventional technology are briefly described hereinafter. Apparently, the drawings in the following description are only some embodiments of the present disclosure, and for those skilled in the art, other embodiments may be obtained based on the provided drawings without creative efforts.

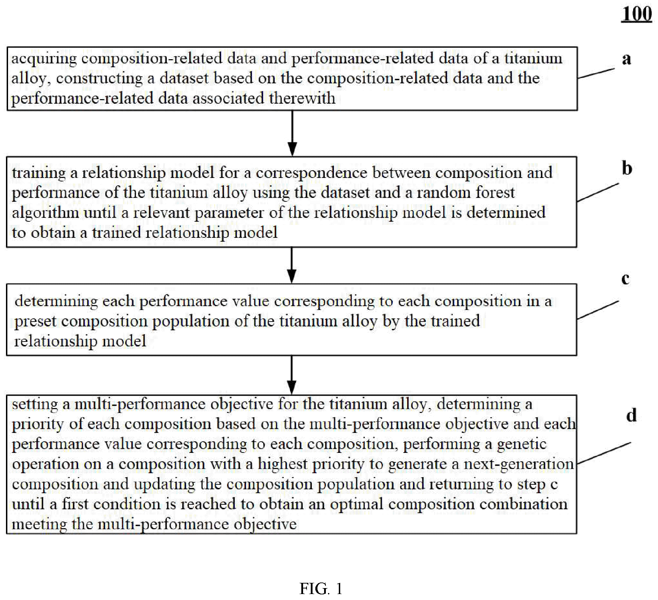

FIG. 1 is a flowchart of a method for optimizing design of titanium alloy composition provided in an embodiment of the present disclosure.

### DETAILED DESCRIPTION

The embodiments of the present disclosure are further described in detail below in conjunction with the accompanying drawings and embodiments. The detailed description and drawings of the following embodiments are used to illustrate the principles of the present disclosure by way of example but are not intended to limit the scope of the present disclosure. The present disclosure may be implemented in many different forms and is not limited to the specific embodiments disclosed herein but includes all technical solutions falling within the scope of the claims.

The present disclosure provides these embodiments so that the present disclosure will be thorough and complete, and to fully convey the scope of the present disclosure to those skilled in the art. It should be noted that, unless otherwise specifically stated, the relative arrangement of components and steps, the composition of materials, numerical expressions, and numerical values set forth in these embodiments should be interpreted as being merely illustrative and not as limiting.

Furthermore, the words "first", "second", and similar words used in the present disclosure does not indicate any order, quantity or importance, but is merely used to distinguish different parts. "Including" or "comprising" and similar words mean that the elements before the word encompass the elements listed after the word, and do not exclude the possibility of encompassing other elements.

All terms used in the present disclosure have the same meaning as understood by those ordinary skilled in the art to which the present disclosure belongs, unless otherwise specifically defined. It should also be understood that terms defined in for example general dictionaries, should be interpreted as having meanings consistent with their meanings in the context of the relevant art, and should not be interpreted with idealized or overly formal meanings, unless explicitly defined as such herein.

The techniques, methods, and equipment known to those ordinary skilled in the relevant fields may not be discussed in detail, but where appropriate, the techniques, methods and equipment should be regarded as part of the specification.

It should be understood that the embodiments of the present disclosure shown in the exemplary embodiments are merely illustrative. Although only a few embodiments are described in detail in the present disclosure, those skilled in the art can easily understand that various modifications are possible without materially departing from the teachings of the present disclosure. Accordingly, all such modifications should be included within the scope of the present disclosure. Other substitutions, modifications, changes and deletions can be made to the design, operating conditions and parameters of the following exemplary embodiments without departing from the gist of the present disclosure.

Referring to FIG. 1, which shows a flowchart of a method for optimizing design of titanium alloy composition and a method for manufacturing a titanium alloy provided in an embodiment of the present disclosure. The method for optimizing design of titanium alloy composition and the method for manufacturing a titanium alloy 100 comprise the following steps.

step a: acquiring composition-related data and performance-related data of a titanium alloy, constructing a dataset based on the composition-related data and the performance-related data associated therewith.

Specifically, the titanium alloy includes TC4, TC11 and the like. The composition-related data of the titanium alloy includes the types and contents of alloying elements and the weights and contents of impurity elements. The alloying elements include aluminum, vanadium, molybdenum, tin and the like, and the impurity elements include iron, oxygen, nitrogen, carbon and the like. The performance-related data of the titanium alloy include mechanical performance-related data, chemical performance-related data, physical performance-related data, and the like, wherein the mechanical performance includes strength, plasticity, toughness, and the like. Regarding titanium alloys, since they are composed of multiple components and possess multiple properties, there is a correspondence between the composition-related data and the performance-related data for different titanium alloys, and multiple composition-related data correspond to multiple performance-related data. Before constructing the dataset, the composition-related data and performance-related data need to be pre-processed to successively complete missing value processing, data format unification, and vectorization processing on the data, for example, regularizing the data using Python, and then constructing the dataset based on the processed data.

step b: training a relationship model for a correspondence between composition and performance of the titanium alloy using the dataset and a random forest algorithm until a relevant parameter of the relationship model is determined to obtain a trained relationship model.

Specifically, the random forest algorithm is an ensemble learning algorithm based on decision trees that can accurately classify samples. The representation of the random forest algorithm belongs to the prior art and is not detailed herein. The dataset is divided into a training set and a testing set. Preferably, the dataset is divided into a training set and a testing set at a ratio of 2:1. The relationship model for the correspondence between the composition and performance of the titanium alloy is trained using the training set and the random forest algorithm, and relevant parameters of the relationship model are optimized using an optimization algorithm until the optimal effect is achieved, thereby determining the final relevant parameters of the relationship model. Wherein, the optimization algorithm includes cross-validation algorithm, grid search algorithm and the like. The determined final relevant parameters of the relationship model include the number of decision trees, (n_estimators), maximum depth (max_depth), minimum number of samples for split (min_samples_split), minimum number of samples required to be at a leaf node (min_samples_leaf), maximum number of features (max_features), and the like. In a specific embodiment, the relationship model for the correspondence between the composition and performance of the titanium alloy is trained with the Skleam package in a Python environment to obtain a trained relationship model, for example, a composition-performance (tensile strength and elongation of TC4 titanium alloy) relationship model.

step c: determining each performance value corresponding to each composition in a preset composition population of the titanium alloy by the trained relationship model.

Specifically, a preset composition population of titanium alloy is obtained by an existing composition generation model. For example, a plurality of composition vectors Xᵢ are randomly generated by a random algorithm, which is represented by a formula of ${x}_{i} = \left[{x}_{i 1},{x}_{i 2},…,{x}_{in}\right]$ (formula 1).

Wherein, a composition vector xᵢₙ represents an n-th element in the composition vector Xᵢ, and a sum of corresponding contents from xᵢ₁ to xᵢₙ is 1. For example, the content of the j-th element in the composition vector Xᵢ is represented as xᵢⱼ, which satisfies ${\sum }_{j = 1}^{n} {x}_{ij} = 100$.

Using the trained relationship model and the aforementioned preset composition population, each performance value corresponding to each composition in the preset composition population is determined.

step d: setting a multi-performance objective for the titanium alloy, determining a priority of each composition based on the multi-performance objective and each performance value corresponding to each composition, performing a genetic operation on a composition with a highest priority to generate a next-generation composition and updating the composition population and returning to step c until a first condition is reached to obtain an optimal composition combination meeting the multi-performance objective.

Specifically, the multi-performance objective of the titanium alloy refers to targets for multiple properties. For example, for the titanium alloy, a tensile strength is greater than or equal to 900 MPa and an elongation is greater than or equal to 20%. Alternatively, for the titanium alloy, a tensile strength is greater than or equal to 800 MPa and an elongation is greater than or equal to 20%. Specifically, the multi-performance objective of the titanium alloy can be flexibly adjusted according to actual requirements and is not limited to the multi-performance objectives listed above.

Specifically, the composition vectors are sorted according to a non-dominated sorting algorithm (NSGA-II). By comparing each performance value corresponding to each composition vector, it is determined which compositions perform better in the optimization process for the multi-performance objective. For any two composition vectors: xᵢ and xⱼ, if they satisfy the following formula 2: *yₖ(xᵢ ≤ yₖ(xⱼ*) and ∃*m* y*ₘ*(xᵢ) < yₘ(xⱼ) (formula 2),
in which k, m∈ {1, 2, 3} k, xᵢ dominates xⱼ. Under the condition of the same domination rank, the compositions are further sorted by calculating a crowding degree. The crowding degree reflects the density of composition points in the objective space. A composition point with a smaller density has a lower crowding degree, and thus a higher priority. The priority corresponding to each composition is determined according to the principle of determining priority rank. Genetic operations are performed on a composition with a highest priority therein by a genetic algorithm, and the crossover operations (for example, generating a new offspring composition xnew (k) by a simulated binary crossover (SBX) operator) and mutation operations (for example, introducing a small perturbation δ on the basis of the offspring composition and performing a polynomial mutation) are sequentially performed to generate a next-generation composition and the composition population is updated. Then, based on the updated composition population, each performance value corresponding to each composition in the updated composition population is determined using the trained relationship model. According to the above steps, iterative updating is performed until a first condition is reached, for example reaching a maximum number of iterations. The composition population is updated with a next-generation composition obtained in the final iteration and the optimal composition combination meeting the multi-performance objective is determined based on the updated composition population.

The method for optimizing design of titanium alloy composition described above eliminates the need for a large number of experiments and comprehensive analysis of experimental data, thereby avoiding cumbersome experimental procedures. Meanwhile, it obviates the need for multiple data preparation and extensive data calculation processes, reducing the complexity of determining titanium alloy composition design via algorithms and improving both the efficiency and accuracy of determining titanium alloy composition design.

According to some embodiments of the present disclosure, step d comprises: comparing a contribution of each performance value corresponding to each composition to the multi-performance objective by a non-dominated sorting algorithm, calculating a crowding degree of each composition, and determining a priority of each composition based on a principle that under the same domination condition, a lower crowding degree corresponds to a higher priority.

According to some embodiments of the present disclosure, step d further comprises performing a crossover operation on a composition with a highest priority according to a formula of ${x}_{new}^{\left(k\right)} = \frac{1 + β}{2} {x}_{p} + \frac{1 - β}{2} {x}_{q}$ (formula 3).
wherein xₚ and x_{q} are two parent compositions, β is a crossover factor generated based on a random number, and X_{new}^{(k)} is a generated pre-mutation next-generation composition; and
performing a mutation operation on the pre-mutation next-generation composition according to a formula of ${x}_{new}^{\left(k\right)} = {x}_{new}^{\left(k\right)} + δ$ (formula 4).
wherein δ is a small perturbation for performing polynomial mutation, and ${x}_{new}^{\left(k\right)}$ is a generated next-generation composition.

To further understand the method for optimizing design of titanium alloy composition of the present disclosure, the following description provides further details in a specific embodiment.

Step 1: Composition-related data and performance-related data of a titanium alloy are collected from laboratories, factories, literature data, and the like.

Step 2: The aforementioned composition-related data and performance-related data are standardized using Python to successively complete missing value processing, data format unification and vectorization processing. Using the library pandas in Python, the data is first comprehensively checked to identify fields or records where missing values are located. According to the characteristics of the data, an appropriate missing value processing strategy is employed, including deleting missing values, filling missing values or marking missing values. Data format is unified with Python, which includes, for example, data type conversion, date and time format processing, and text data processing. Finally, all features are vectorized, and a dataset is constructed based on the processed data.

Step 3: The dataset is divided into a training set and a testing set at a ratio of 2:1. High-precision random forest machine learning model is trained using the training set to establish a composition-performance (tensile strength, yield strength and elongation of TC4 titanium alloy) relationship model. To ensure the rationality of the model, cross-validation and grid search methods are used to determine relevant parameters of the random forest model. The relevant parameters include the number of decision trees, (n_estimators), maximum depth (max_depth), minimum number of samples for split (min_samples_split), minimum number of samples required to be at a leaf node (min_samples_leaf), maximum number of features (max_features), and the like. The training process described above is completed using the Sklearn package in a Python environment to obtain a composition-performance (tensile strength and elongation of TC4 titanium alloy) relationship model for subsequent invocation by the non-dominated sorting genetic algorithm.

Step 4: A multi-performance objective is set, for example, setting the tensile strength of the titanium alloy to be greater than or equal to 900 MPa, and the elongation to be greater than or equal to 20%. An initial composition population is generated using a random algorithm, for example, randomly generating 400 composition vectors Xᵢ represented by formula 1, which will not be repeated here.

Step 5: The composition vectors are sorted according to a non-dominated sorting algorithm (NSGA-II). The non-dominated sorting judges which compositions perform better in the optimization process for the multi-performance objective by comparing each performance value corresponding to each composition vector. For any two composition vectors xᵢ and x_{j,} if formula 2 is satisfied, under the condition of the same dominance degree, the compositions are further sorted by calculating crowding degree. The crowding degree reflects the density of composition points in the objective space. A composition point with a smaller density has a lower crowding degree, and thus a higher priority. Specifically, the composition points are first projected into a two-dimensional space, and Delaunay triangulation method is used to perform triangulation on each composition point to calculate the area of the triangle enclosing the point. A larger area indicates a lower density around the point, and thus a lower crowding degree, and a higher priority. Delaunay Triangulation is an algorithm in computational geometry, which is used to divide a set of points on a plane into a series of non-overlapping triangles, such that the collection of these triangles completely covers the convex hull of the original set of points.

Step 6: The priority of each composition is determined based on the sorting results, and a composition with an optimal performance (i.e., composition top-ranked in non-dominated sorting and crowding degree sorting) is selected and subjected to genetic operations, including crossover operations and mutation operations, wherein the formula for the crossover operation is represented by formula 3, and the mutation operation is represented by formula 4, which will not be repeated here.

Step 7: A cycle of step 6 and step 7 is repeated. After generating each new generation composition, re-prediction and sorting are sequentially performed using the relationship model and the non-dominated sorting algorithm, and then genetic operations are performed to generate a next-generation composition, until a preset number of iteration steps gₘₐₓ is reached.

Step 8: In a final generation, compositions that meet the performance requirements are screened out. These compositions perform well in the multi-objective optimization and satisfy the preset multi-performance objective.

According to a second aspect of the present disclosure, provided is a method for manufacturing a titanium alloy, comprising preparing a plurality of raw materials according to the method for optimizing design of titanium alloy composition according to any one of items described above, pre-processing the plurality of raw materials, placing them into a smelting furnace, controlling a relevant parameter of a smelting process and then casting and shaping to obtain a titanium alloy.

The various embodiments of the present disclosure have been described herein in detail. To avoid obscuring the concept of the present disclosure, some details well known in the art have not been described. Those skilled in the art can fully understand how to implement the technical solutions disclosed herein based on the above description.

Although some specific embodiments of the present disclosure have been described in detail by way of examples, those skilled in the art should understand that the above examples are for illustration only and are not intended to limit the scope of the present disclosure. Those skilled in the art should understand that modifications may be made to the above embodiments or equivalent replacements may be made to some technical features without departing from the scope and spirit of the present disclosure. In particular, the technical features mentioned in the various embodiments may be combined in any manner, provided there is no structural conflict.

## Claims

1. A method for optimizing design of titanium alloy composition, comprising
step a: acquiring composition-related data and performance-related data of a titanium alloy, constructing a dataset based on the composition-related data and the performance-related data associated therewith,
step b: training a relationship model for a correspondence between composition and performance of the titanium alloy using the dataset and a random forest algorithm until a relevant parameter of the relationship model is determined to obtain a trained relationship model,
step c: determining each performance value corresponding to each composition in a preset composition population of the titanium alloy by the trained relationship model,
step d: setting a multi-performance objective for the titanium alloy, determining a priority of each composition based on the multi-performance objective and each performance value corresponding to each composition, performing a genetic operation on a composition with a highest priority to generate a next-generation composition and updating the composition population and returning to step c until a first condition is reached to obtain an optimal composition combination meeting the multi-performance objective.

2. The method for optimizing design of titanium alloy composition according to claim 1, wherein the multi-performance objective for the titanium alloy includes a tensile strength of the titanium alloy being greater than or equal to 900 MPa and an elongation of the titanium alloy being greater than or equal to 20%.

3. The method for optimizing design of titanium alloy composition according to claim 2, wherein step d comprises:
comparing a contribution of each performance value corresponding to each composition to the multi-performance objective by a non-dominated sorting algorithm, calculating a crowding degree of each composition, and determining a priority of each composition based on a principle that under the same domination condition, a lower crowding degree corresponds to a higher priority.

4. The method for optimizing design of titanium alloy composition according to claim 3, wherein step d further comprises performing a crossover operation on a composition with a highest priority according to a formula of ${x}_{new}^{\left(k\right)} = \frac{1 + β}{2} {x}_{p} + \frac{1 - β}{2} {x}_{q}$,
wherein xₚ and x_{q} are two parent compositions, β is a crossover factor generated based on a random number, and X_{new}^{(k)} is a generated pre-mutation next-generation composition; and
performing a mutation operation on the pre-mutation next-generation composition according to a formula of ${x}_{new}^{\left(k\right)} = {x}_{new}^{\left(k\right)} + δ$,
wherein δ is a small perturbation for performing polynomial mutation, and ${x}_{new}^{\left(k\right)}$ is a generated next-generation composition.

5. The method for optimizing design of titanium alloy composition according to claim 4, wherein step d further comprises until a maximum number of iterations is reached, updating the composition population with a next-generation composition obtained in a final iteration and determining an optimal composition combination meeting the multi-performance objective based on the updated composition population.

6. The method for optimizing design of titanium alloy composition according to claim 1, wherein the preset composition population is generated by a random algorithm, and the composition population comprises a plurality of composition vectors Xᵢ represented by a formula of **x***ᵢ* = [*x*_{*i*1}, *x*_{*i*2}, ... , *xᵢₙ*],
wherein a composition vector xᵢₙ represents an n-th element in the composition vector Xᵢ, and a sum of corresponding contents from xᵢ₁ to xᵢₙ is 1.

7. The method for optimizing design of titanium alloy composition according to claim 1, wherein step b comprises:
dividing the dataset into a training set and a testing set at a ratio of 2:1, training a relationship model for a correspondence between the composition and the performance of the titanium alloy using the training set and a random forest algorithm, optimizing the relationship model by a cross-validation and a grid search, determining a relevant parameter of the relationship model, verifying the accuracy of the relationship model using the testing set, to obtain the trained relationship model if the verification is passed.

8. The method for optimizing design of titanium alloy composition according to claim 1, wherein step a comprises:
acquiring the composition-related data and the performance-related data of the titanium alloy, performing missing value processing, data format unification, and vectorization processing on the composition-related data and the performance-related data that are associated with each other, and constructing a dataset based on the processed data.

9. The method for optimizing design of titanium alloy composition according to claim 1, wherein the relevant parameter of the relationship model includes the number of decision trees, a maximum depth, a minimum number of samples for split, a minimum number of samples required to be at a leaf node and a maximum number of features.

10. A method for manufacturing a titanium alloy, comprising:
preparing a plurality of raw materials according to the method for optimizing design of titanium alloy composition according to any one of claims 1 to 9,
pre-processing the plurality of raw materials, placing them into a smelting furnace, controlling a relevant parameter of a smelting process and then casting and shaping to obtain a titanium alloy.
